# EUROPEAN PATENT SPECIFICATION

(11) **EP 3 283 052 B1**
(45) Date of publication and mention of the grant of the patent: **19.02.2020**
(21) Application number: 16717119.8
(22) Date of filing: 13.04.2016
(51) Int. Cl.: A61K 8/73, A61Q 19/08

(54) **SKIN CARE COMPOSITIONS COMPRISING MICROFIBRILLATED CELLULOSE**
HAUTPFLEGEZUSAMMENSETZUNGEN MIT MIKROFIBRILLIERTER CELLULOSE
COMPOSITIONS DE SOINS POUR LA PEAU CONTENANT DE LA CELLULOSE MICROFIBRILLÉE

(30) Priority: 13.04.2015 EP 15001048
(43) Date of publication of application: 21.02.2018
(73) Proprietor: Borregaard AS, 1721 Sarpsborg (NO)
(72) Inventor: BLELL, Rebecca, 1701 Sarpsborg (NO); VOLD, Inger Mari Nygård, 1701 Sarpsborg (NO); ØVREBØ, Hans Henrik, 1701 Sarpsborg (NO); GONERA, Antje, 1540 Vestby (NO)
(74) Representative: Tostmann, Holger Carl
(86) International application number: PCT/EP2016/058149
(87) International publication number: WO 2016/166179

(56) References cited:
- WO-A1-2007/091942
- WO-A1-2013/031030
- WO-A1-2015/180844
- WO-A2-2007/078857
- US-A- 4 374 702
- US-A1- 2012 156 270
- DATABASE WPI Week 201443 Thomson Scientific, London, GB; AN 2014-L35102 XP002743879, & WO 2014/088072 A1 (NIPPON PAPER IND CO LTD) 12 June 2014 (2014-06-12)

## Description

The present invention relates to the use of microfibrillated cellulose as an anti-wrinkle agent. The present invention also relates to a skin care composition comprising at least one microfibrillated cellulose and to a method for preparing the same.

### Background of the Invention

Anti-wrinkle and/or anti-aging creams are in increasing demand for everyday use, also under aspects of skin protection. It has therefore become important for cosmetics manufacturers to include anti-wrinkle ingredients in basic cream formulations for creams, ointments and the like.

Skin moisturizers and sunscreens also may be formulated to provide benefits as anti-aging creams.

Examples of anti-wrinkle ingredients that may be used in anti-aging creams are:
- Retinol;
- Epidermal growth factor;
- Alpha hydroxy acids (AHAs) and beta hydroxy acids or other chemical peels;
- Peptides, such as Matryxil and copper peptides;
- Coenzyme Q10;
- Argireline (also known as acetyl hexapeptide-3);
- Anti-oxidants;
- Vitamin C.

The above listed ingredients are of synthetic origin and do not satisfy increasing demands for products of natural origin.

Therefore, it is an object of the present invention to provide a new type of anti-wrinkle agent, which is of natural origin. In particular, a less expensive alternative to the anti-wrinkle agents known from the art is seen as desirable.

Cellulose-based materials are used in anti-aging masks or products, in principle, usually as a swellable media or as the support used to apply the anti-wrinkle ingredient on the desired part of human skin.

US 2004/0156811A1 describes microcrystalline cellulose as an enhancing agent for wrinkle-hiding in decorative skin cosmetics.

Microfibrillar cellulose has been used in personal care products (e.g. body washes, hand soaps and conditioners) mostly as a rheology modifier (as a thickener, a suspending aid and/or as a gel forming agent). WO2013031030A1 describes MFC as a gel forming agent in cosmetic sheets. WO2013094077A1 describes microfibrillar cellulose as building material for cosmetic sheets. EP2307100A2 describes MFC as a rheology modifier in liquid cleanser compositions.

EP1144103A2 describes nanofibrils of amorphous cellulose as an emulsifying agent and/or a dispersion stabilizer. HU9903102A2 describes nanofibrils of amorphous cellulose as a viscosity modifier in cosmetics and detergent products.

### Summary of the Invention

The above mentioned object and other objects are achieved by the teaching of the present invention. Surprisingly, it has been found that microfibrillated cellulose has a pronounced anti-wrinkle effect on human skin.

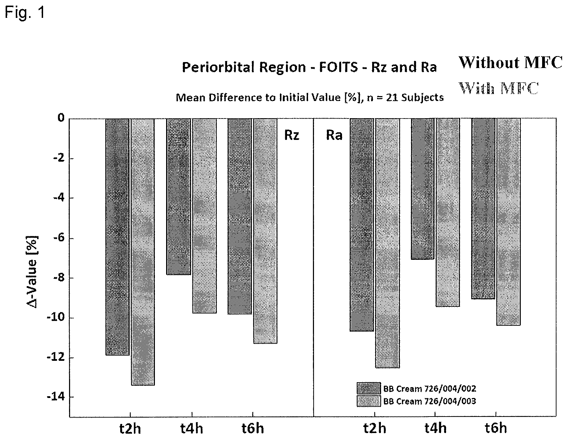

Fast optical in vivo topometry of human skin (FOITS) measurement revealed a pronounced anti-wrinkle effect of microfibrillated cellulose formulated in a beauty balm ('BB') cream on human skin in as short a time span as 2 hours. This effect was also observed at 4 hours and at 6 hours in a kinetic FOITS measurement. The BB cream comprising MFC did show a significantly increased anti-wrinkle effect, at all three time points, compared to the respective BB cream without MFC, as illustrated in Figure 1.

Without wishing to be bound by theory, it is believed that the positive effect observed in this experiment might be due to the fact that MFC is a high molecular weight filling agent.

The cellulose fibrils in the cream are believed to fill in the external surface roughness or wrinkles, resulting in an immediate anti-wrinkle effect on skin. Alternatively or additionally, the microfibrillated cellulose might act as a film former on the skin, stretching it slightly and thereby reducing wrinkles.

Another characteristic of MFC that might contribute to its anti-wrinkle effect is it comparatively high water holding capacity (water retention capacity) which might result in hydration of the skin. Preferably, the microfibrillated cellulose has a water holding capacity of at least 40, preferably at least 50, preferably at least 60, preferably at least 70, preferably at least 75, preferably at least 80, preferably at least 90, further preferably at least 100 as measured in accordance with the method described herein. Without wishing to be bound by theory, it is believed that improved water retention is due to the specific morphology of the MFC fibrils/fibril bundles.

Thus, in a first aspect, the present invention relates to the use of microfibrillated cellulose as an anti-wrinkle agent, particularly for use on human skin, as defined in the appended set of claims.

In a second aspect, the present invention relates to a skin care composition comprising at least one microfibrillated cellulose, said at least one microfibrillated cellulose being characterized by a water holding capacity of at least 40, preferably at least 50, preferably at least 60, preferably at least 70, preferably at least 75, preferably at least 80, preferably at least 90, further preferably at least 1 00, as defined in the appended set of claims.

The present invention further relates to a method of preparing a skin care composition comprising mixing at least one fatty phase and at least one aqueous phase thereby forming an oil in water (O/W) emulsion, said at least one fatty phase or said at least one aqueous phase comprising at least one microfibrillated cellulose, said at least one microfibrillated cellulose being characterized by a water holding capacity of at least 40, preferably at least 50, preferably at least 60, preferably at least 70, preferably at least 75, preferably at least 80, preferably at least 90, further preferably at least 100, as defined in the appended set of claims.

Preferred embodiments of the present invention are the subject-matter of the dependent claims.

### Detailed Description of the Invention

In the following, the invention is described with reference to the enclosed figure, wherein:
- **Figure 1**: shows the effect of a BB cream comprising MFC on wrinkles of human skin after 2 hours, 4 hours and 6 hours compared to the effect of a BB cream without MFC.

Advantages, preferred embodiments and possible applications of the present invention are described in the following.

Microfibrillated cellulose (MFC) as used within the claimed composition and in the meaning of the present invention relates to cellulose fibers of any conceivable origin. In particular, MFC according to the present invention relates to cellulose in fiber form that has been subjected to a mechanical treatment in order to increase the fibers' specific surface and to reduce their size in terms of cross-section and of length, wherein said size reduction leads to a fiber diameter in the nanometer range and a fiber length in the micrometer range.

MFC (also known as "microfibrillar cellulose", "reticulated cellulose", "superfine cellulose" or as "cellulose nanofibrils", among others) is prepared from cellulose fibers which are defibrillated using high pressure and/or high mechanical force. In the cellulose starting material (typically present as a "cellulose pulp") no, or at least not a significant or not even a noticeable portion of individualized and "separated" cellulose fibrils can be found. By contrast, in MFC, individual fibrils and fibril bundles can be easily discerned by way of conventional optical microscopy. Sometimes, these fibrils and bundles of fibrils are also described as "(micro)fibrils". In accordance with the present invention, any reference to "fibrils" also includes bundles of such fibrils. Microfibrillated cellulose is described e.g. in US 4 481 077, US 4 374 702 and US 4 341 807. According to US 4 374 702 ("Turbak"), microfibrillated cellulose has properties distinguishable from previously known celluloses.

Due to its large surface area and a high aspect ratio (length to width ratio), microfibrillated cellulose is believed to have a good ability to form rigid networks. In solution, MFC typically forms a highly viscous gel-like dispersion with shear thinning properties. The large surface area of the MFC and the high amount of accessible hydroxyl groups cause MFC to typically have a high water holding capacity.

MFC in accordance with "Turbak" is produced by passing a liquid suspension of cellulose through a small diameter orifice in which the suspension is subjected to a large pressure drop and a high velocity shearing action followed by a high velocity decelerating impact and repeating the passage of said suspension through the orifice until the cellulose suspension becomes a substantially stable suspension.

The process converts the cellulose into microfibrillated cellulose without substantial chemical change of the cellulose starting material.

An improved process for obtaining particularly homogeneous MFC is described in WO 2007/091942.

The microfibrillated cellulose in accordance with the present invention may be produced according to any process known in the art. Preferably, said method comprises at least one mechanical step and at least one homogenizing step. The mechanical pretreatment step preferably is or comprises a refining step. The method can also comprise a chemical pretreatment step. One example of such pretreatment step might be oxidation of the hydroxyl groups on the surface of the microfibrils to carboxylic acids. Negative charges of the carboxylic groups cause repulsion between the microfibrils which aids the defibrillation of the cellulose.

The purpose of the mechanical pretreatment step is to "beat" the cellulose pulp in order to increase the accessibility of the cell walls, i.e. increase the surface area and therefore to increase the water retention value. In the refiner that is preferably used in the mechanical pretreatment step, one or two rotating disk(s) is/are employed, i.e. the cellulose pulp slurry is subjected to shear forces.

Prior to the mechanical or chemical pretreatment step, or in between the mechanical or chemical pretreatment steps or as the mechanical pretreatment step, enzymatic (pre)treatment of the cellulose pulp is an optional additional step that may be preferred for some applications. In regard to enzymatic pretreatment in conjunction with microfibrillating cellulose, the respective content of WO 2007/091942 can be consulted.

Most preferably, the microfibrillated cellulose is prepared and obtainable by the process disclosed in WO 2015/180844. This process comprises at least the following steps:
(a) subjecting a cellulose pulp to at least one **mechanical pretreatment step**;
(b) subjecting the mechanically pretreated cellulose pulp of step (a) to a **homogenizing step,** which results in fibrils and fibril bundles of reduced length and diameter vis-à-vis the cellulose fibers present in the mechanically pretreated cellulose pulp of step (a), said step (b) resulting in microfibrillated cellulose;
   wherein the homogenizing step (b) involves compressing the cellulose pulp from step (a) and subjecting the cellulose pulp to a pressure drop, by expanding the cellulose through at least one orifice, providing a pressure drop between a volume segment, preferably a chamber, that is located upstream of said orifice, and another volume segment, preferably a chamber, that is located downstream of said orifice, area,
   wherein said pressure drop is at least 1000 bar, preferably more than 2000 bar, preferably more than 2500 bar, further preferably more than 3000 bar, and
   wherein the cellulose fibrils are subjected to a turbulent flow regime in said volume segment, preferably a chamber, that is located downstream of said orifice.

The mechanical pretreatment step preferably is or comprises a refining step. A refiner that is preferably used in the mechanical pretreatment step comprises at least one rotating disk. Therein, the cellulose pulp slurry is subjected to shear forces between the at least one rotating disk and at least one stationary disk. Preferably, the refining step is repeated at least 5 times, preferably at least 10 times, further preferably at least 30 times.

In the homogenizing step (b), which is to be conducted after the (mechanical) pretreatment step, the cellulose pulp slurry from step (a) is passed through a homogenizer at least once, preferably at least two times.

This process yields microfibrillated cellulose with a comparatively high specific surface area and a water holding capacity (water retention capacity) of at least 75, particularly at least 80 or even at least 100 as determined using the method described below.

Furthermore, the viscous properties of a gel-like dispersion of the MFC prepared by the process disclosed in WO 2015/180844 in organic solvents are improved vis-à-vis MFCs known from the art, in particular in regard to the zero shear viscosity. A gel-like dispersion of the microfibrillated cellulose produced by the above described method disclosed in WO 2015/180844 in polyethylene glycol (PEG400) is characterized by a zero shear viscosity, η₀, of at least 5000 Pa•s, particularly at least 6000 Pa•s or even at least 7000 Pa•s, as measured at a solids content of the MFC of 0.65% and 35% H₂O. The G'ₗᵢₙ value of a gel-like dispersion comprising the thus prepared microfibrillated cellulose in polyethylene glycol (PEG400) is more than 250 Pa, particularly more than 350 Pa, as measured at a solids content of the MFC of 0.65% and 35% H₂O.

The microfibrillated cellulose preferably has a high aspect ratio which corresponds to the ratio of the length of the microfibril bundle to its diameter (L/D). To this end, the pulp slurry is preferably passed through a homogenizer (a high-pressure homogenizer or a low-pressure homogenizer) and subjected to a pressure drop by forcing the pulp slurry between opposing surfaces, preferably orifices. The term "orifice" means an opening or a nozzle or a valve contained in a homogenizer suitable for homogenizing cellulose.

In particular embodiments, the length and/or the diameter of the cellulose fibrils and fibril bundles of the microfibrillated cellulose are reduced vis-à-vis the respective length and diameter of the cellulose fibers and fiber bundles making up the cellulose that was used as a starting point.

In particular embodiments, the average MFC fibril length is from 100 nm to 50 µm, preferably from 500 nm to 25 µm, more preferably from 1 µm to 10 µm, most preferably from 3 µm to 10 µm.

In particular embodiments, the average MFC fibril diameter is from 1 nm to 500 nm, preferably from 5 nm to 100 nm, more preferably from 10 nm to 50 nm, most preferably from 10 nm to 30 nm.

In particular embodiments, the average MFC fibril aspect ratio is comparatively high.

Fibril length and fibril diameter are determined by imaging using Atomic Force Microscopy and/or Scanning Electron Microscopy.

In particular embodiments, the MFC has a comparatively high specific surface area. Within the context of the present application, the term "specific surface area" refers to the total surface area of an MFC material per unit of mass.

In particular embodiments, the microfibrillated cellulose has a water holding capacity (water retention capacity) of at least 40, preferably at least 50, preferably at least 60, preferably at least 70, preferably at least 75, preferably at least 80, preferably at least 90, further preferably at least 100. The water holding capacity describes the ability of the MFC to retain water within the MFC structure and this again relates to the accessible surface area. According to the invention, the water holding capacity is measured by diluting an MFC sample to a 0.3% solids content in water so that the total volume of the suspension is 100 ml and then mixing the suspension with IKA T25 digital Ultra Turrax dispersing device at 8000 rpm for 4 min. Then, the suspension is centrifuged at 1000 G for 15 minutes. The clear water phase is separated from the sediment and the sediment is weighed. The water holding capacity is given as (mV/mT)-1 where mV is the weight of the wet sediments and mT is the weight of dry MFC analyzed.

In order to conduct (comparative) measurements of the water holding capacity, preferably, deionized water should be used, and a minimum value for the conductivity (ion strength) of 35 µS/cm should be observed, or, in the alternative a value in the range of from 35 µS/cm to 2000 µS/cm. If the conductivity of the 0.3% dispersion is below 35 µS/cm, adjustment by adding of sodium chloride is performed.

In particular embodiments, a dispersion of the microfibrillated cellulose in polyethylene glycol (PEG400) has a zero shear viscosity, η₀, of at least 5000 Pa•s, preferably at least 6000 Pa•s, further preferably at least 7000 Pa•s, as measured at a solids content of the MFC of 0.65% and 35% H₂O.

In particular embodiments, a gel-like dispersion of the microfibrillated cellulose in polyethylene glycol (PEG400) has a G'ₗᵢₙ value of more than 250 Pa, preferably more than 350 Pa, as measured at a solids content of the MFC of 0.65% and 35% H₂O.

The rheological characterization of the MFC dispersions is performed with PEG400 as the solvent. "PEG400" is a polyethylene glycol with a molecular weight between 380 and 420 g/mol and is widely used in pharmaceutical applications and therefore commonly known and available.

The rheological properties, in particular zero shear viscosity, as disclosed and claimed herein are measured on a rheometer of the type *Anton Paar* Physica MCR 301 at a temperature of 25 °C, using a "plate-plate" geometry (diameter: 50mm). Rheological measurements may be performed as an oscillating measurement (amplitude sweep) to evaluate the degree of structure in the dispersions (complex viscosity and storage modulus G'ₗᵢₙ) or as rotational viscosity measurement, in which case the viscosity is measured as a function of the shear rate to evaluate the viscosity at rest (shear forces → 0), as well as the shear thinning properties of the dispersions.

Without wishing to be bound by theory, it is believed that the bundles of fibrils might have a matting effect on skin hiding its flaws. This may allow the skin care compositions to give the skin a matte finish and to conceal small flaws or blemishes thereby resulting in an anti-wrinkle effect.

In principle, the raw material for the cellulose microfibrils may be any cellulosic material, in particular cellulose originating from wood, annual plants, cotton, flax, straw, ramie, sugar cane bagasse, certain algae, jute, sugar beet, citrus fruits, waste from the food processing industry or energy crops or cellulose of bacterial or animal origin, e.g. from tunicates.

In particular embodiments, wood-based materials are used as raw materials, either hardwood or softwood or both (in mixtures). Further preferably softwood is used as a raw material, either one kind or mixtures of different soft wood types.

The term MFC, in accordance with the present invention, encompasses a single kind of microfibrillated cellulose as well as a mixture of structurally different microfibrillated celluloses, such as a mixture of unmodified and chemically and/or physically modified microfibrillated cellulose.

The microfibrillated cellulose in accordance with the present invention may be unmodified with respect to its functional group or may be physically modified or chemically modified, preferably resulting in neutral or negatively charged groups on the microfibril's surface, or both. In one embodiment, the MFC, in accordance with the present invention, is substantially free of cationic substituents.

The preferred embodiment of chemical modification of the surface of the cellulose microfibrils in the present invention is preferably achieved by various reactions of surface functional groups of the cellulose microfibrils and more particularly of the hydroxyl functional groups, preferably resulting in neutral or negatively charged groups on the microfibril surface. Examples of such reactions are oxidation, silylation reactions, etherification reactions, condensations with isocyanates, alkoxylation reactions with alkylene oxides or condensation or substitution reactions with glycidyl derivatives. Chemical modification may take place before or after the defibrillation step. Thus, in particular embodiments, the MFC is chemically modified by oxidation, silylation, etherification, condensation with isocyanates, alkoxylation with alkylene oxides, condensation with glycidyl derivatives, substitution with glycidyl derivatives or any combination thereof.

The cellulose microfibrils may also be modified by a substantially 'physical' method, which does not involve the formation of chemical bonds to a substantial extend, in particular, either by adsorption at the surface, or by spraying, coating or encapsulation of the microfibril. Preferred modified microfibrils can be obtained by physical adsorption of at least one compound. The microfibrils can be modified by physical adsorption of at least one amphiphilic compound (surfactant).

In one embodiment, microfibrils are modified by physical adsorption of at least one non-ionic surfactant. EP2 408 857 describes processes of preparing surface modified MFC. The physical modification of the MFC surface may take place before or after the defibrillation step.

Physical modification of the cellulose microfibril surface may occur prior to the mechanical pretreatment step, between the mechanical pretreatment step and the defibrillation step or after the defibrillation step.

The microfibrillated cellulose (MFC) according to the present invention may be subjected to at least one dewatering and/or drying step. The at least one drying step is preferably selected from freeze-, spray- or roller-drying, drying in a convection oven, flash drying or the like. 'Never-dried' microfibrillated cellulose may also be used and the microfibrillated cellulose used in the present invention might have a dry content ranging from 0.1 %-100% before it is added to a skin care composition.

In particular embodiments, the dry content of the MFC is from 0.1% to 100%, preferably from 0.2% to 40%, more preferably from 0.5% to 25%, even more preferably from 1% to 20%, before the MFC is added to the composition.

Within the context of the present application, the term "anti-wrinkle agent" refers to a substance having wrinkle reducing effects on human skin, meaning that the depth of at least a portion of the wrinkles that are contacted with the anti-wrinkle agent is reduced to some extend for a certain period of time. For example, the average wrinkle depth may be reduced by 0.2%, 0.5%, 1%, 2%, 5%, 10%, 15; 20% or more instantly or after 1 h, 2 h, 3 h, 6 h, 12 h or 24 h after application of the anti-wrinkle agent. The wrinkle reducing effect may persist for 2 h, 4 h, 6 h, 8 h, 10 h, 12 h, 24 h, 36 h or longer. For example, a 'wrinkle reducing' effect exists, if a reduction of the average wrinkle depth by at least 0.2% is observed 2 h after the application of the anti-wrinkle agent and persists for at least a further 2 h.

According to one embodiment, the microfibrillated cellulose is provided in a skin care composition. These skin care compositions are preferably used for topical application onto the skin. The cosmetic composition is preferably semi-solid at room temperature.

The MFC comprising composition may be in any suitable form commonly used in cosmetics. In particular embodiments, the composition is selected from a cream, a lotion, a paste, an emulsion, a gel, a foundation, a serum, and an ointment. Particularly preferred is the addition of microfibrillated cellulose to beauty balm creams. In accordance with the present invention, the term "beauty balm cream" or "BB cream" refers to an all-in-one facial cosmetic product which may replace multiple simultaneously used cosmetic products. BB creams have moisturizing effects, sun protection effects and some also 'anti-aging' properties. They are generally suitable to replace skin moisturizers, foundation as well as sunscreens.

Accordingly, in particular embodiments, the skin care composition further comprises at least one skin moisturizer and at least one UV filter.

Within the context of the present application, the term "moisturizer" refers to a dermatologically compatible agent that helps to restore moisture or to retain moisture in the skin upon application. Suitable moisturizers may be selected from, but are not limited to: naturally occurring skin/membrane lipids and sterols (steroid alcohols), artificial or natural oils, humectants, emollients, and lubricants.

Within the context of the present application, the term "UV filter" refers to an agent able to block, absorb and/or reflect UV rays. Suitable UV filters may be selected from, but are not limited to, Zinc Oxide, Titanium Dioxide, Avobenzone, Tinosorb S, Tinosorb M, Mexoryl SX, Mexoryl XL, Helioplex, Octinoxate, Octocrylene, Oxybenzone, Octisalate, Homosalate, Uvinul T 150, Cinoxate, Aminobenzoic acid, Padimate O, Ensulizole, Dioxybenzone, Meradimate, Sulisobenzone, Trolamine salicylate, Enzacamene, Bisdisulizole Disodium, Uvinul A Plus, Uvasorb HEB, Parsol SLX and Amiloxate.

The skin care composition may include further components or ingredients which are compatible with human skin. The ingredients may be chosen according to the selected application of the product and may be chosen from, but are not limited to, excipients, surfactants, emulsifiers, thickeners, vitamins, fats, oils, waxes, humectants, UV filters, preservatives, water, alcohol, perfumes, anti-foaming agents, foam stabilizers, polymers, electrolytes, organic solvents, silicone derivatives and coloring agents.

The formulation of pharmaceutically-acceptable dermatological and cosmetic preparations, in principle, is known in the art.

In particular embodiments, the concentration of the microfibrillated cellulose in the skin care composition is from 0.04% to 25% by weight, preferably from 0.1 to 10% by weight, more preferably from 0.2 to 5% by weight, most preferably from 0.5 to 2% by weight.

In a second aspect, the present invention relates to a skin care composition comprising at least one microfibrillated cellulose, said at least one microfibrillated cellulose being characterized by a water holding capacity of at least 40, preferably at least 50, preferably at least 60, preferably at least 70, preferably at least 75, preferably at least 80, preferably at least 90, further preferably at least 100, as defined in the appended claims.

The skin care composition is an oil in water (O/W) emulsion, particularly a beauty balm cream. In particular embodiments, the skin care composition further comprises titanium dioxide as UV filter.

In a third aspect, the present invention relates to a method of preparing a skin care composition comprising mixing at least one fatty phase and at least one aqueous phase thereby forming an oil in water (O/W) emulsion, said at least one fatty phase or said at least one aqueous phase comprising at least one microfibrillated cellulose, said at least one microfibrillated cellulose being characterized by a water holding capacity of at least of at least 40, preferably at least 50, preferably at least 60, preferably at least 70, preferably at least 75, preferably at least 80, preferably at least 90, further preferably at least 100, as defined in the appended claims.

In particular embodiments, the method of preparing a skin care composition is a method of preparing a beauty balm cream. In particular embodiments, the beauty balm cream further comprises titanium dioxide. In particular embodiments, the method of preparing a beauty balm cream comprises preparing an oil in water (O/W) emulsion by mixing a fatty phase (phase A in table 1), titanium dioxide for sun protection (phase B in table 1) and an aqueous phase comprising at least one microfibrillated cellulose (phase C in table 1).

### Examples

### FOITS test measurements carried out on BB creams with and without MFC

MFC was tested for its anti-wrinkle properties in a kinetic measurement test using FOITS as the measurement method. In order to perform the test, 1% (dry material) MFC was formulated in a Beauty Balm BB cream. A paired comparison against Placebo formulation without MFC on 20 female volunteers was carried out. Formulation work and FOITS measurements were carried out in the laboratories of Institute Dr. Schrader, Max-Planck-Straße 6, D-37603 Holzminden - Germany.

### Preparation of the BB creams

The BB creams used in these tests, BB cream 726/004/002 and BB cream 726/004/003, contain the ingredients listed in Table 1.

The Beauty balm creams were prepared on a laboratory scale of approximately 500 g using standard stirrers and dissolvers. Titanium dioxide was dispersed in the hot fatty phase by a high shear Ultra Turrax™. The MFC was dispersed at room temperature in the aqueous phase using a dissolver. The aqueous phase was then heated to 80°C. After emulsification, the emulsion was finally homogenized in a Homozenta™ emulsifying machine.

**Table 1.**

| Pos. | Raw Material | INCI (Eu) | Supplier | % [w/w] 726/004/002 | % [w/w] 726/004/003 |
|---|---|---|---|---|---|
| Phase A | | | | | |
| 1 | Amphisol K | Potassium Cetyl Phosphate | Nordmann Rassmann | 2.20 | 2.20 |
| 2 | Lanette O | Cetearyl Alcohol | BTC Europe GmbH | 2.00 | 2.00 |
| 3 | Tegin M Pellets | Glyceril Stearate | Goldschmid t Evonik | 1.00 | 1.00 |
| 4 | Softisan 100 | Hydrogenated Coco glycerides | Sasol Germany GmbH | 2.00 | 2.00 |
| 5 | Eutanol G | Octyldodecanol | BTC Europe GmbH | 5.00 | 5.00 |
| 6 | Isopropylpalmitat (IPP) | Isopropyl Palmitate | BTC Europe GmbH | 8.00 | 8.00 |
| 7 | Neo Heliopan 303 (600154) | Octorylene | Symrise | 2.00 | 2.00 |
| 8 | all-rac-alpha-Tocopherylacetat (Art. 500952) | Tocopheryl Acetate | Merck | 0.50 | 0.50 |
| 9 | Euxyl PE 9010 | Phenoxyethanol, Ethylhexylglycerin, Tocopherol | Schülke & Mayr | 1.00 | 1.00 |

| Phase B | | | | | |
|---|---|---|---|---|---|
| 10 | Eusolex T-AVO (105335) | Titanium Dioxide (Nano), Silica | Merck KGaA | 3.00 | 3.00 |
| 11 | Kronos 1171 (Titandioxid, 260 nm) | CI 77891 | Kronos Titan | 3.00 | 3.0 |
| 12 | Symcolor Eisenoxidgelb 656835 | CI 77492 | Symrise | 0.70 | 0.70 |
| 13 | Dragocolor Eisenoxidrot E172 656836 | CI 77491 | Symrise | 0.16 | 0.16 |
| 14 | Dragocolor Eisenoxidschwarz 656837 | CI 77499 | Symrise | 0.08 | 0.08 |

| Phase C | | | | | |
|---|---|---|---|---|---|
| | | | | | |
| 15 | Water, demin. | AQUA | | 64.91 | 54.91 |
| 16 | Edeta BD | Disodium EDTA | BTC Europe GmbH | 0.05 | 0.05 |
| 17 | Glycerin Ph. Eur. 85% | Glycerin, Aqua | Azelis | 4.00 | 4.00 |
| 18 | Keltrol CG-SFT | Xanthan Gum | Rahn AG | 0.40 | 0.40 |
| 19 | MFC-Paste m/Phenoxyethanol 10% active matter | | Borregaard | -- | 10.00 |
| | | | | 100.00 | 100.00 |

Microfibrillated cellulose can be prepared as described herein.

The cream was applied to the skin and the FOITS measurements of the Periorbital region were recorded.

### Fast Optical in Vivo Topometry of human skin (FOITS)

The three-dimensional skin structure was determined by FOITS measurements. The FOITS method is a non-contact method of three-dimensional wrinkle analysis based on an optical measuring technique (fringe projection) for three-dimensional data collection providing an objective touch free, non-invasive, in vivo assessment of skin microtopography. The FOITS methodology allows for full 3D-visualisation of the anti-wrinkle or anti-aging effect and statistical analysis of roughness parameters Rz and Ra according to the expanded version of DIN 4768.

After measuring the initial situation (t0), the measurements were repeated after two (t2h), four (t4h) and six hours (t6h).

### Periorbital Region - FOITS - Rz and Ra - Summary and Statistics

Rz represents a measure of the peak to valley size of the largest features (largest wrinkles). Ra is a measure of the overall residual difference from profile centerline (overall roughness).

Table 2 and Figure 1 show the mean values obtained for Rz and Ra at initial time of application of both BB creams, after 2h, after 4h and after 6h. The statistical analysis of the data shows a significant difference between the values obtained by the Bb cream without MFC and that with MFC.

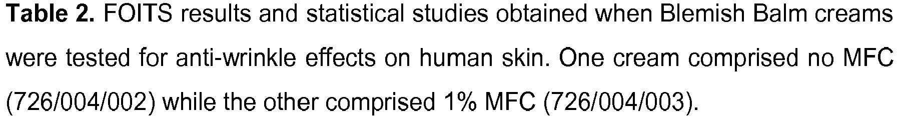

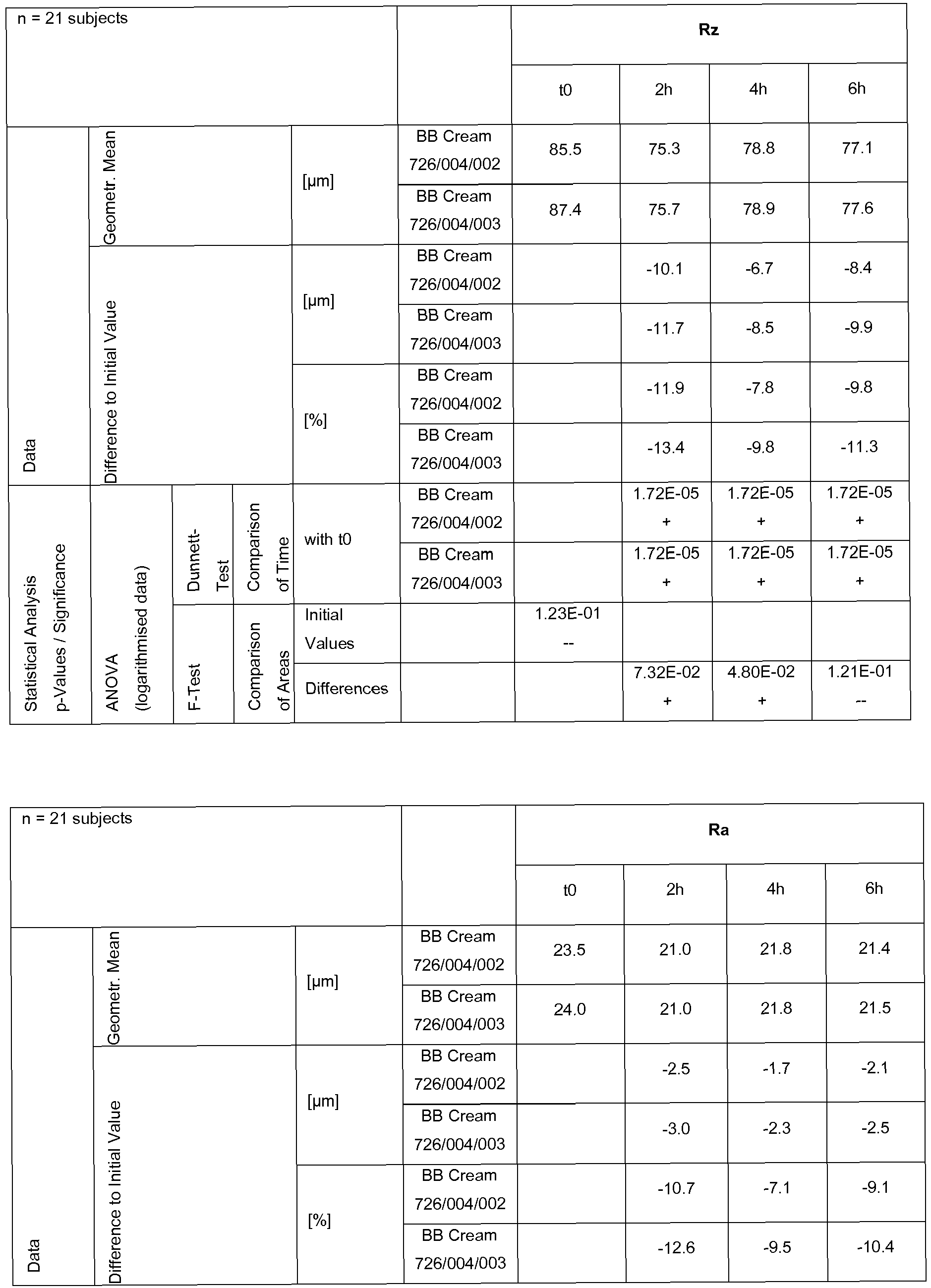

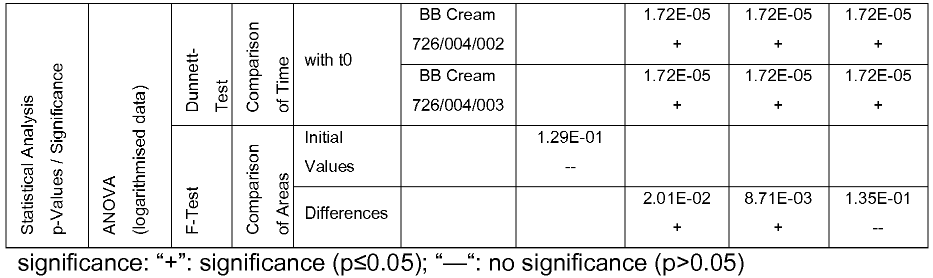

BB cream 726/004/003 (with MFC) and the control 726/004/002 (without MFC) both showed a significant anti-wrinkle effect after 2 h, 4 h and 6 h compared to time t0 because both BB creams comprise several other care components (lipids) and moisturizers (water, glycerol etc.).

The BB cream with MFC showed a significantly increased anti-wrinkle effect after 2 h, 4h as well as 6 h compared to the BB cream without MFC. Figure 1 clearly illustrates the pronounced anti-wrinkle effect of the cream with MFC compared to the cream without MFC.

## Claims

1. Use of microfibrillated cellulose (MFC) as an anti-wrinkle-agent on human skin, wherein the average MFC fibril length is from 100 nm to 50 µm.

2. The use according to claim 1, wherein the MFC is provided in a skin care composition, preferably selected from a cream, a lotion, a paste, an emulsion, a gel, a foundation, a serum, and an ointment.

3. The use according to claim 1 or 2, wherein the MFC is an unmodified MFC or a chemically modified MFC, preferably having neutral or negatively charged substituents, or a physically modified MFC or any combination thereof.

4. The use according to any one of the preceding claims, wherein the average MFC fibril length is from 500 nm to 25 µm, preferably from 1 µm to 10 µm, more preferably from 3 µm to 10 µm.

5. The use according to any one of the preceding claims, wherein the average MFC fibril diameter is from 1 nm to 500 nm, preferably from 5 nm to 100 nm, more preferably from 10 nm to 50 nm, most preferably from 10 nm to 30 nm.

6. The use according to any one of the preceding claims, where the water holding capacity of the MFC is at least 40, preferably at least 50, preferably at least 60, preferably at least 70, preferably at least 75, preferably at least 80, preferably at least 90, further preferably at least 100, wherein the water holding capacity is measured by diluting the MFC sample to a 0.3% solids content in water so that the total volume of the suspension is 100 ml , mixing the suspension with IKA T25 digital Ultra Turrax dispersing device at 8000 rpm for 4 min, centrifuging the suspension at 1000 G for 15 minutes, separating the clear water phase from the sediment and weighing the sediment, wherein the water holding capacity is given as (mV/mT)-1 where mV is the weight of the wet sediments and mT is the weight of dry MFC analyzed.

7. The use according to any one of the preceding claims, wherein the MFC is unmodified MFC.

8. The use according to any one of the preceding claims, wherein the MFC is chemically modified by oxidation, silylation, etherification, condensation with isocyanates, alkoxylation with alkylene oxides, condensation with glycidyl derivatives, substitution with glycidyl derivatives or any combination thereof.

9. The use according to any one of the preceding claims, wherein the MFC is physically modified by physical absorption of at least one amphiphilic compound, preferably by a non-ionic amphiphilic compound.

10. The use according to any one of the preceding claims, wherein the MFC is free of cationic substituents.

11. The use according to any one of claims 2 to 10, wherein the dry content of the MFC is from 0.1% to 100%, preferably from 0.2% to 40%, more preferably from 0.5% to 25%, more preferably from 1% to 20%, before the MFC is added to the composition.

12. The use according to any one of claims 2 to 11, wherein the concentration of the MFC in the skin care composition is from 0.04% to 25% by weight, preferably from 0.1 to 10% by weight, more preferably from 0.2 to 5% by weight, most preferably from 0.5 to 2% by weight.

13. The use according to any one of claims 2 to 12, wherein the composition further comprises at least one skin moisturizer and at least one UV filter.

14. Skin care composition comprising at least one microfibrillated cellulose in an aqueous phase, and at least one fatty phase, thereby forming an oil in water (O/W) emulsion, said at least one microfibrillated cellulose being **characterized by** a water holding capacity of at least 40, preferably at least 50, preferably at least 60, preferably at least 70, preferably at least 75, preferably at least 80, preferably at least 90, further preferably at least 100, wherein the water holding capacity is measured by diluting the MFC sample to a 0.3% solids content in water so that the total volume of the suspension is 100 ml, mixing the suspension with IKA T25 digital Ultra Turrax dispersing device at 8000 rpm for 4 min, centrifuging the suspension at 1000 G for 15 minutes, separating the clear water phase from the sediment and weighing the sediment, wherein the water holding capacity is given as (mV/mT)-1 where mV is the weight of the wet sediments and mT is the weight of dry MFC analyzed,
wherein said at least one microfibrillated cellulose is unmodified, physically modified or chemically modified by oxidation, silylation, condensation with isocyanates, alkoxylation with alkylene oxides, condensation with glycidyl derivatives, substitution with glycidyl derivatives or any combination thereof.

15. A method of preparing a skin care composition comprising mixing at least one fatty phase and at least one aqueous phase thereby forming an oil in water (O/W) emulsion, said at least one fatty phase or said at least one aqueous phase comprising at least one microfibrillated cellulose, said at least one microfibrillated cellulose being **characterized by** a water holding capacity of at least 40, preferably at least 50, preferably at least 60, preferably at least 70, preferably at least 75, preferably at least 80, preferably at least 90, further preferably at least 100, wherein the water holding capacity is measured by diluting the MFC sample to a 0.3% solids content in water so that the total volume of the suspension is 100 ml, mixing the suspension with IKA T25 digital Ultra Turrax dispersing device at 8000 rpm for 4 min, centrifuging the suspension at 1000 G for 15 minutes, separating the clear water phase from the sediment and weighing the sediment, wherein the water holding capacity is given as (mV/mT)-1 where mV is the weight of the wet sediments and mT is the weight of dry MFC analyzed,
wherein said at least one microfibrillated cellulose is unmodified, physically modified or chemically modified by oxidation, silylation, condensation with isocyanates, alkoxylation with alkylene oxides, condensation with glycidyl derivatives, substitution with glycidyl derivatives or any combination thereof.

## Patentansprüche

1. Verwendung von mikrofibrillierter Cellulose (MFC) als Antifaltenmittel auf menschlicher Haut, wobei die durchschnittliche MFC-Fibrillenlänge 100 nm bis 50 µm beträgt.

2. Verwendung nach Anspruch 1, wobei die MFC in einer Hautpflegezusammensetzung vorhanden ist, die vorzugsweise ausgewählt ist aus einer Creme, einer Lotion, einer Paste, einer Emulsion, einem Gel, einer Grundierung, einem Serum und einer Salbe.

3. Verwendung nach Anspruch 1 oder 2, wobei die MFC eine unmodifizierte MFC oder eine chemisch modifizierte MFC, vorzugsweise mit neutralen oder negativ geladenen Substituenten, oder eine physikalisch modifizierte MFC oder eine Kombination davon ist.

4. Verwendung nach einem der vorhergehenden Ansprüche, wobei die durchschnittliche MFC-Fibrillenlänge 500 nm bis 25 µm, bevorzugt 1 µm bis 10 µm, bevorzugter 3 µm bis 10 µm, beträgt.

5. Verwendung nach einem der vorhergehenden Ansprüche, wobei der mittlere MFC-Fibrillendurchmesser 1 nm bis 500 nm, bevorzugt 5 nm bis 100 nm, bevorzugter 10 nm bis 50 nm, ganz besonders bevorzugt 10 nm bis 30 nm, beträgt.

6. Verwendung nach einem der vorhergehenden Ansprüche, wobei das Wasserhaltevermögen der MFC mindestens 40, vorzugsweise mindestens 50, vorzugsweise mindestens 60, vorzugsweise mindestens 70, vorzugsweise mindestens 75, vorzugsweise mindestens 80, vorzugsweise mindestens 90, weiter bevorzugt mindestens 100, beträgt,
wobei das Wasserhaltevermögen bestimmt wird, indem die MFC-Probe in Wasser auf einen Feststoffgehalt von 0,3% verdünnt wird, so dass das Gesamtvolumen der Suspension 100 mL beträgt, die Suspension mit einer digitalen IKA T25 Ultra Turrax-Dispergiervorrichtung bei 8000 U/min für 4 min gemischt wird, die Suspension bei 1000 G für 15 Minuten zentrifugiert wird, die klare Wasserphase von dem Sediment getrennt wird und das Sediment gewogen wird, wobei das Wasserhaltevermögen in (mV/mT)-1 angegeben ist, worin mV das Gewicht des nassen Sediments und mT das Gewicht der trockenen zu analysierenden MFC ist.

7. Verwendung nach einem der vorhergehenden Ansprüche, wobei die MFC eine unmodifizierte MFC ist.

8. Verwendung nach einem der vorhergehenden Ansprüche, wobei die MFC durch Oxidation, Silylierung, Veretherung, Kondensation mit Isocyanaten, Alkoxylierung mit Alkylenoxiden, Kondensation mit Glycidylderivaten, Substitution mit Glycidylderivaten oder einer Kombination davon chemisch modifiziert ist.

9. Verwendung nach einem der vorhergehenden Ansprüche, wobei die MFC durch physikalische Absorption von mindestens einer amphiphilen Verbindung, vorzugsweise einer nichtionischen amphiphilen Verbindung, physikalisch modifiziert ist.

10. Verwendung nach einem der vorhergehenden Ansprüche, wobei die MFC frei von kationischen Substituenten ist.

11. Verwendung nach einem der Ansprüche 2 bis 10, wobei der Trockengehalt der MFC bevor die MFC der Zusammensetzung zugesetzt wird 0,1% bis 100%, bevorzugt 0,2% bis 40%, bevorzugter 0,5% bis 25%, bevorzugter 1% bis 20%, beträgt.

12. Verwendung nach einem der Ansprüche 2 bis 11, wobei die Konzentration der MFC in der Hautpflegezusammensetzung 0,04 Gew.-% bis 25 Gew.-%, vorzugsweise 0,1 bis 10 Gew.-%, weiter bevorzugt 0,2 bis 5 Gew.-%, am bevorzugtesten 0,5 bis 2 Gew.-%, beträgt.

13. Verwendung nach einem der Ansprüche 2 bis 12, wobei die Zusammensetzung ferner mindestens einen Hautbefeuchter und mindestens einen UV-Filter umfasst.

14. Hautpflezusammensetzung, umfassend mindestens eine mikrofibrillierte Cellulose in einer wässrigen Phase und mindestens eine Fettphase, wodurch eine Öl-in-Wasser (O/W)-Emulsion gebildet wird, wobei die mindestens eine mikrofibrillierte Cellulose **gekennzeichnet ist durch** ein Wasserhaltevermögen von mindestens 40, bevorzugt mindestens 50, bevorzugt mindestens 60, bevorzugt mindestens 70, bevorzugt mindestens 75, bevorzugt mindestens 80, bevorzugt mindestens 90, weiter bevorzugt mindestens 100,
wobei das Wasserhaltevermögen bestimmt wird, indem die MFC-Probe in Wasser auf einen Feststoffgehalt von 0,3% verdünnt wird, so dass das Gesamtvolumen der Suspension 100 mL beträgt, die Suspension mit einer digitalen IKA T25 Ultra Turrax-Dispergiervorrichtung bei 8000 U/min für 4 min gemischt wird, die Suspension bei 1000 G für 15 Minuten zentrifugiert wird, die klare Wasserphase von dem Sediment getrennt wird und das Sediment gewogen wird, wobei das Wasserhaltevermögen in (mV/mT)-1 angegeben ist, worin mV das Gewicht des nassen Sediments und mT das Gewicht der trockenen zu analysierenden MFC ist,
wobei die mindestens eine mikrofibrillierte Cellulose unmodifiziert, physikalisch modifiziert oder chemisch modifiziert ist mittels Oxidation, Silylierung, Kondensation mit Isocyanaten, Alkoxylierung mit Alkylenoxiden, Kondensation mit Glycidylderivaten, Substitution mit Glycidylderivaten oder einer Kombination davon.

15. Verfahren zur Herstellung einer Hautpflegezusammensetzung, umfassend das Mischen von mindestens einer Fettphase und mindestens einer wässrigen Phase, wodurch eine ÖI-in-Wasser(O/W)-Emulsion gebildet wird, wobei die mindestens eine Fettphase oder die mindestens eine wässrige Phase mindestens eine mikrofibrillierte Cellulose umfasst, wobei die mindestens eine mikrofibrillierte Cellulose **gekennzeichnet ist durch** ein Wasserhaltevermögen von mindestens 40, bevorzugt mindestens 50, bevorzugt mindestens 60, bevorzugt mindestens 70, bevorzugt mindestens 75, bevorzugt mindestens 80, bevorzugt mindestens 90, weiter bevorzugt mindestens 100,
wobei das Wasserhaltevermögen bestimmt wird, indem die MFC-Probe in Wasser auf einen Feststoffgehalt von 0,3% verdünnt wird, so dass das Gesamtvolumen der Suspension 100 mL beträgt, die Suspension mit einer digitalen IKA T25 Ultra Turrax-Dispergiervorrichtung bei 8000 U/min für 4 min gemischt wird, die Suspension bei 1000 G für 15 Minuten zentrifugiert wird, die klare Wasserphase von dem Sediment getrennt wird und das Sediment gewogen wird, wobei das Wasserhaltevermögen in (mV/mT)-1 angegeben ist, worin mV das Gewicht des nassen Sediments und mT das Gewicht der trockenen zu analysierenden MFC ist,
wobei die mindestens eine mikrofibrillierte Cellulose unmodifiziert, physikalisch modifiziert oder chemisch modifiziert ist mittels Oxidation, Silylierung, Kondensation mit Isocyanaten, Alkoxylierung mit Alkylenoxiden, Kondensation mit Glycidylderivaten, Substitution mit Glycidylderivaten oder einer Kombination davon.

## Revendications

1. Utilisation de cellulose microfibrillée (MFC) en tant qu'agent antirides sur peau humaine, dans laquelle la longueur moyenne de fibrille de MFC est comprise entre 100 nm et 50 µm.

2. Utilisation selon la revendication 1, dans laquelle la MFC est fournie dans une composition de soin de la peau, préférentiellement choisie parmi une crème, une lotion, une pâte, une émulsion, un gel, un fond de teint, un sérum, et une pommade.

3. Utilisation selon la revendication 1 ou 2, dans laquelle la MFC est une MFC non modifiée ou une MFC modifiée chimiquement, préférentiellement ayant des substituants neutres ou chargés négativement, ou une MFC modifiée physiquement ou n'importe quelle combinaison de celles-ci.

4. Utilisation selon l'une quelconque des revendications précédentes, dans laquelle la longueur moyenne de fibrille de MFC est comprise entre 500 nm et 25 µm, préférentiellement entre 1 µm et 10 µm, plus préférentiellement entre 3 µm et 10 µm.

5. Utilisation selon l'une quelconque des revendications précédentes, dans laquelle le diamètre moyen de fibrille de MFC est compris entre 1 nm et 500 nm, préférentiellement entre 5 nm et 100 nm, plus préférentiellement entre 10 nm et 50 nm, encore plus préférentiellement entre 10 nm et 30 nm.

6. Utilisation selon l'une quelconque des revendications précédentes, où la capacité de rétention d'eau de la MFC est d'au moins 40, préférentiellement au moins 50, préférentiellement au moins 60, préférentiellement au moins 70, préférentiellement au moins 75, préférentiellement au moins 80, préférentiellement au moins 90, plus préférentiellement au moins 100, dans laquelle la capacité de rétention d'eau est mesurée par dilution de l'échantillon de MFC à une teneur en matières solides de 0.3% dans l'eau de telle sorte que le volume total de la suspension est de 100 ml, mélange de la suspension avec le dispositif de dispersion IKA T25 digital Ultra Turrax à 8000 rpm pendant 4 min, centrifugation de la suspension à 1000 G pendant 15 minutes, séparation de la phase aqueuse claire du sédiment et pesée du sédiment, dans laquelle la capacité de rétention d'eau est donnée par la formule (mV/mT)-1 où mV est la masse des sédiments humides et mT est la masse de MFC sèche analysée.

7. Utilisation selon l'une quelconque des revendications précédentes, dans laquelle la MFC est une MFC non modifiée.

8. Utilisation selon l'une quelconque des revendications précédentes, dans laquelle la MFC est modifiée chimiquement par oxydation, silylation, éthérification, condensation avec des isocyanates, alcoxylation avec des oxydes d'alkylène, condensation avec des dérivés glycidyliques, substitution avec des dérivés glycidyliques ou n'importe quelle combinaison de ceux-ci.

9. Utilisation selon l'une quelconque des revendications précédentes, dans laquelle la MFC est modifiée physiquement par absorption physique d'au moins un composé amphiphile, préférentiellement par un composé amphiphile non-ionique.

10. Utilisation selon l'une quelconque des revendications précédentes, dans laquelle la MFC ne contient pas de substituants cationiques.

11. Utilisation selon l'une quelconque des revendications 2 à 10, dans laquelle la teneur en matière sèche de la MFC est comprise entre 0.1% et 100%, préférentiellement entre 0.2% et 40%, plus préférentiellement entre 0.5% et 25%, plus préférentiellement entre 1% et 20%, avant l'ajout de la MFC à la composition.

12. Utilisation selon l'une quelconque des revendications 2 à 11, dans laquelle la concentration de la MFC dans la composition de soin de la peau est comprise entre 0.04% et 25% en poids, préférentiellement entre 0.1% et 10% en poids, plus préférentiellement entre 0.2% et 5% en poids, plus préférentiellement entre 0.5% et 2% en poids.

13. Utilisation selon l'une quelconque des revendications 2 à 12, dans laquelle la composition comprend en outre au moins un hydratant pour la peau et au moins un filtre UV.

14. Composition de soin de la peau comprenant au moins une cellulose microfibrillée dans une phase aqueuse, et au moins une phase grasse, formant ainsi une émulsion d'huile dans l'eau (O/W), ladite au moins une cellulose microfibrillée étant **caractérisée par** une capacité de rétention d'eau d'au moins 40, préférentiellement au moins 50, préférentiellement au moins 60, préférentiellement au moins 70, préférentiellement au moins 75, préférentiellement au moins 80, préférentiellement au moins 90, plus préférentiellement au moins 100, dans laquelle la capacité de rétention d'eau est mesurée par dilution de l'échantillon de MFC à une teneur en matières solides de 0.3% dans l'eau de telle sorte que le volume total de la suspension est de 100 ml, mélange de la suspension avec le dispositif de dispersion IKA T25 digital Ultra Turrax à 8000 rpm pendant 4 min, centrifugation de la suspension à 1000 G pendant 15 minutes, séparation de la phase aqueuse claire du sédiment et pesée du sédiment, dans laquelle la capacité de rétention d'eau est donnée par la formule (mV/mT)-1 où mV est la masse des sédiments humides et mT est la masse de MFC sèche analysée,
dans laquelle ladite au moins une cellulose microfibrillée est non modifiée, modifiée physiquement ou modifiée chimiquement par oxydation, silylation, condensation avec des isocyanates, alcoxylation avec des oxydes d'alkylène, condensation avec des dérivés glycidyliques, substitution avec des dérivés glycidyliques ou n'importe quelle combinaison de ceux-ci.

15. Procédé visant à la préparation d'une composition de soin de la peau comprenant un mélange d'au moins une phase grasse et au moins une phase aqueuse formant ainsi une émulsion d'huile dans l'eau (O/W), ladite au moins une phase grasse ou ladite au moins une phase aqueuse comprenant au moins une cellulose microfibrillée, ladite au moins une cellulose microfibrillée étant **caractérisée par** une capacité de rétention d'eau d'au moins 40, préférentiellement au moins 50, préférentiellement au moins 60, préférentiellement au moins 70, préférentiellement au moins 75, préférentiellement au moins 80, préférentiellement au moins 90, plus préférentiellement au moins 100, dans laquelle la capacité de rétention d'eau est mesurée par dilution de l'échantillon de MFC à une teneur en matières solides de 0.3% dans l'eau de telle sorte que le volume total de la suspension est de 100 ml, mélange de la suspension avec le dispositif de dispersion IKA T25 digital Ultra Turrax à 8000 rpm pendant 4 min, centrifugation de la suspension à 1000 G pendant 15 minutes, séparation de la phase aqueuse claire du sédiment et pesée du sédiment, dans laquelle la capacité de rétention d'eau est donnée par la formule (mV/mT)-1 où mV est la masse des sédiments humides et mT est la masse de MFC sèche analysée,
dans laquelle ladite au moins une cellulose microfibrillée est non modifiée, modifiée physiquement ou modifiée chimiquement par oxydation, silylation, condensation avec des isocyanates, alcoxylation avec des oxydes d'alkylène, condensation avec des dérivés glycidyliques, substitution avec des dérivés glycidyliques ou n'importe quelle combinaison de ceux-ci.
